# EUROPEAN PATENT APPLICATION

(11) **EP 4 085 800 A1**
(43) Date of publication of application: **09.11.2022**
(21) Application number: 22171549.3
(22) Date of filing: 04.05.2022
(51) Int. Cl.: A47G 1/00, A45D 44/00

(54) **VOICE RECOGNITION BASED SMART MIRROR DEVICE**

(30) Priority: 07.05.2021 KR 20210059570; 29.04.2022 KR 20220053192
(71) Applicant: Icon AI Inc., Seocho-gu Seoul 06770 (KR)
(72) Inventor: SHIN, Min Young, 06600 Seoul (KR)
(74) Representative: BCKIP Part mbB

(57) **Abstract**

A voice recognition based smart mirror device (10) includes: a plurality of arms (110,120,130) rotatably connected to each other; and a mirror unit (200) rotatably mounted to one of the plurality of arms. The mirror unit includes: a body part (210); a joint part (220) through which the body part is pivotably connected to the one of the plurality of arms; a mirror part (230) rotatably mounted to the body part; a display module part mounted on a front surface of the body part to output an image, the display module part (240) serving to sense a touch signal of a user; a recognition part for detecting a voice signal of the user; and a controller configured to control the display module part in accordance with the voice signal or the touch signal.

## Description

### TECHNICAL FIELD

The present disclosure relates to a voice recognition based smart mirror device.

### BACKGROUND

In general, mirrors have long been mainly used as a means for humans to check their appearance.

Recently, there has been proposed a smart mirror display device which not only allows humans to check their appearance in real time, but also provides various information through a display.

However, since a conventional smart mirror display device is used in a state that it is fixed on the wall, it is inconvenient for the user to use the mirror display device at a close distance while adjusting it at a desired height and angle, which arises a problem in that the utilization in various spaces is low.

### (Prior Art Document)

Korean Patent Application Publication No. 10-2019-0068146

### SUMMARY

In view of the above, the present disclosure provides a voice recognition based smart mirror device capable of being easily mounted on a wall.

In accordance with an embodiment of the present disclosure, there is a voice recognition based smart mirror device including: a plurality of arms rotatably connected to each other; a mirror unit rotatably mounted to one of the plurality of arms; a speaker installed in at least one of the plurality of arms to output a sound; and a bracket to be fixedly installed on a wall and configured to selectively mount one of the plurality of arms, wherein the mirror unit includes: a body part; a joint part through which the body part is pivotably connected to the one of the plurality of arms; a mirror part rotatably mounted to the body part; a display module part mounted on a front surface of the body part to output an image, the display module part serving to sense a touch signal of a user; a recognition part for detecting a voice signal of the user; and a controller configured to control the display module part in accordance with the voice signal or the touch signal.

The mirror unit may further include: a display module part mounted on a front surface of the body part and configured to detect a touch signal of the user, and the controller may be configured to control the display module part based on the touch signal.

The voice recognition based smart mirror device may further include: an actuator for rotating at least one of the plurality of arms, wherein the actuator shifts a position of the mirror unit under the control of the controller to allow the mirror unit to be directed to a face of the user at various angles.

The actuator may be linked to a voice assistant of the controller so that the position of the mirror unit is controlled in response to the voice signal of the user.

The mirror unit may further include a mirror motor for rotating the mirror part with respect to the body part about an axis extending upward from the body part, and the controller may be configured to control the mirror motor so that the mirror part and the body part are positioned on the same plane when the plurality of arms are rotated so that arm angles between the plurality of arms are smaller than a predetermined reference angle.

The plurality of arms may include a first arm, a second arm, and a third arm, the predetermined reference angle includes a first reference angle between the first arm and the third arm and a second reference angle between the second arm and the third arm, and at least one of the first reference angle and the second reference angle is an acute angle.

The voice recognition based smart mirror device may further include: an angle sensor for detecting arm angles between the plurality of arms that are changed when the plurality of arms are rotated with respect to each other.

The body part may include: a body ring with a through hole in which the mirror part is mounted; a body support provided under the body ring to support the display module part; and a body shaft that connects the mirror part and the body support to allow the mirror part to rotate in the through hole.

A curved edge surface may be formed at an upper end portion of the body support, the curved edge surface being inclined forward and curved along an edge of the body support so as to be convex upward.

The plurality of arms may include: a first arm to which the mirror unit is mounted; a second arm to be fixedly installed on a wall surface through a fixing bracket; a third arm including a speaker therein and having a sound slit for outputting a sound of the speaker; a first rotation shaft that connects one end portion of the first arm and one end portion of the third arm in an up-down direction to allow the first arm and the third arm to be rotated relative to each other; and a second rotation shaft that connects one end portion of the second arm and the other end portion of the third arm in the up-down direction to allow the second arm and the third arm to be rotated relative to each other.

The plurality of arms may include: a first arm to which the mirror unit is mounted; a second arm to be fixedly installed on a wall surface through a fixing bracket; a third arm including a speaker therein and having a sound slit for outputting a sound of the speaker; a first link connecting one end portion of the first arm and one end portion of the third arm in a front-rear direction to link the first arm and the third arm; and a second link connecting one end portion of the second arm and the other end portion of the third arm in the front-rear direction to link the second arm and the third arm.

When the mirror unit is separated from the arm or docked to the arm in a state in which the display module part or the speaker outputs a sound, the controller may control the display module part and the speaker to maintain the output of the sound.

The mirror unit may further include: a request input part configured to receive a request from a user with respect to a biometric information measuring device for measuring biometric information of the user; an artificial intelligence platform service module configured to recognize an operation corresponding to the received request among operations to be operated by the biometric information measuring device and generate a control command corresponding to the recognized operation; and a transmitting part configured to transmit the generated control command to the biometric information measuring device.

According to embodiments of the present disclosure, when using the voice recognition based smart mirror of the present disclosure, the user can analyze his/her face from various angles, and it is possible to mount the smart mirror device on a wall easily.

In addition, according to embodiments of the present disclosure, a user can move the smart mirror device with respect to the wall surface through his/her voice signal. Further, the user can obtain through the smart mirror device various information such as weather, news, music, security, beauty, healthcare, and the like if necessary, and control the smart mirror device in conjunction with smart home, loT or the like.

### BRIEF DESCRIPTION OF THE DRAWINGS

FIG. 1 is a perspective view illustrating a front side of a voice recognition based smart mirror device according to a first embodiment of the present disclosure.
FIG. 2 is a perspective view illustrating a rear side of the voice recognition based smart mirror device according to the first embodiment of the present disclosure.
FIG. 3 is a cross-sectional view taken along line "III-III" of FIG. 1.
FIG. 4 is a perspective view illustrating an operating state of the voice recognition based smart mirror device according to the first embodiment of the present disclosure.
FIG. 5 is a state diagram illustrating a state in which the mirror unit is rotated in FIG. 4.
FIG. 6 is a block diagram illustrating a control flow of the voice recognition based smart mirror device according to the first embodiment of the present disclosure.
FIG. 7 is a perspective view illustrating a front side of a voice recognition based smart mirror device according to a second embodiment of the present disclosure.
FIG. 8 is a perspective view illustrating a rear side of the voice recognition based smart mirror device according to the second embodiment of the present disclosure.
FIG. 9 is a cross-sectional view taken along line "IX-IX" of FIG. 7.
FIG. 10 is a perspective view illustrating an operating state of the voice recognition based smart mirror device according to the second embodiment of the present disclosure.
FIG. 11 is a state diagram illustrating a state in which the mirror unit is rotated in FIG. 10.
FIG. 12 is a perspective view illustrating a front side of a voice recognition based smart mirror device according to a third embodiment of the present disclosure.
FIG. 13 is a state diagram illustrating a state in which the mirror unit is rotated in FIG. 12.
FIG. 14 is a state view illustrating a state in which a mirror unit of a voice recognition based smart mirror device according to the present disclosure is docked to an arm or a main body.
FIGS. 15 to 17 are perspective views illustrating a state in which the display module and the speaker are removed from the voice recognition based smart mirror device of the present disclosure.
FIGS. 18 to 20 are perspective views illustrating the voice recognition based smart mirror device of the present disclosure in which the mirror unit has a circular shape.
FIGS. 21 to 32 are perspective views showing the voice recognition based smart mirror device of the present disclosure to which an arm rotatable up and down is applied.
FIGS. 33 and 34 illustrate a state in which a biometric information measuring device is mounted on the smart mirror device of the present disclosure.
FIG. 35 is a block diagram illustrating a control flow of the voice recognition based smart mirror device including an angle sensor.
FIGS. 36 is a perspective view showing an operating state of the voice recognition based smart mirror device including the angle sensor.

### DETAILED DESCRIPTION

Hereinafter, specific embodiments for implementing a spirit of the present disclosure will be described in detail with reference to the drawings.

In describing the present disclosure, detailed descriptions of known configurations or functions may be omitted to clarify the present disclosure.

When an element is referred to as being 'connected' to, 'supported' by, 'accessed' to, 'supplied' to, 'transferred' to, or 'contacted' with another element, it should be understood that the element may be directly connected to, supported by, accessed to, supplied to, transferred to, or contacted with another element, but that other elements may exist in the middle.

The terms used in the present disclosure are only used for describing specific embodiments, and are not intended to limit the present disclosure. Singular expressions include plural expressions unless the context clearly indicates otherwise.

Further, in the present disclosure, it is to be noted that expressions, such as the upper side and the lower side, are described based on the illustration of drawings, but may be modified if directions of corresponding objects are changed. For the same reasons, some components are exaggerated, omitted, or schematically illustrated in the accompanying drawings, and the size of each component does not fully reflect the actual size.

Terms including ordinal numbers, such as first and second, may be used for describing various elements, but the corresponding elements are not limited by these terms. These terms are only used for the purpose of distinguishing one element from another element.

In the present specification, it is to be understood that the terms such as "including" are intended to indicate the existence of the certain features, areas, integers, steps, actions, elements, combinations, and/or groups thereof disclosed in the specification, and are not intended to preclude the possibility that one or more other certain features, areas, integers, steps, actions, elements, combinations, and/or groups thereof may exist or may be added.

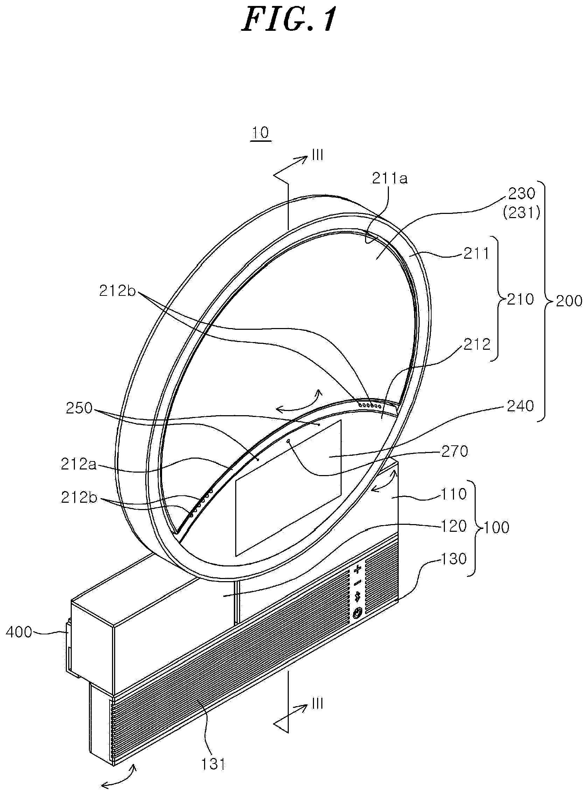

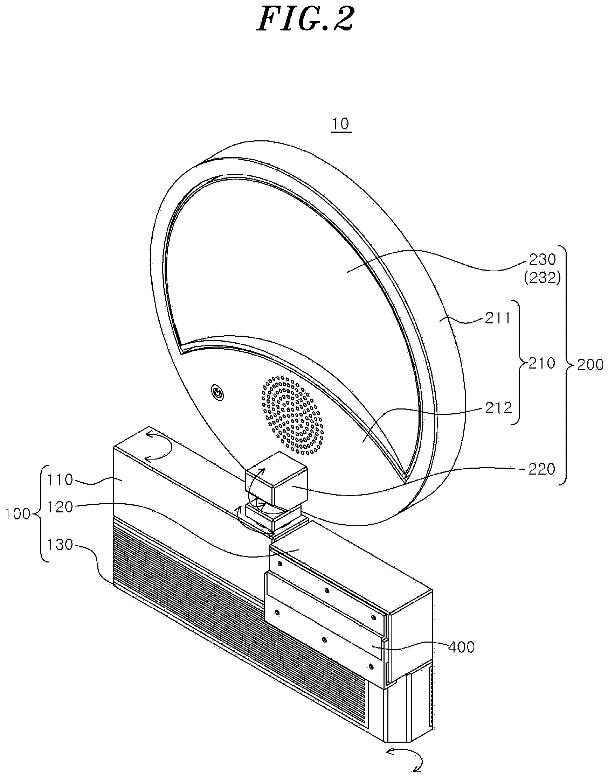

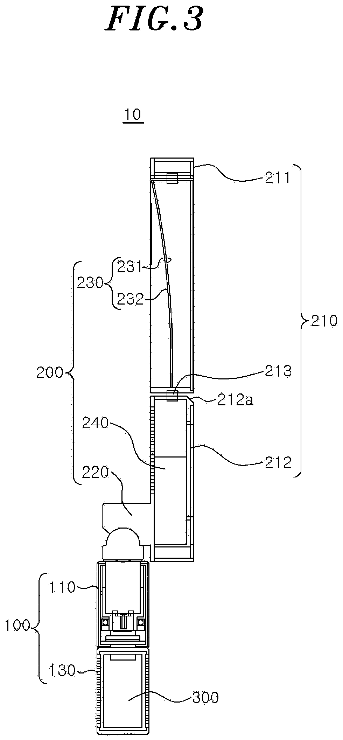

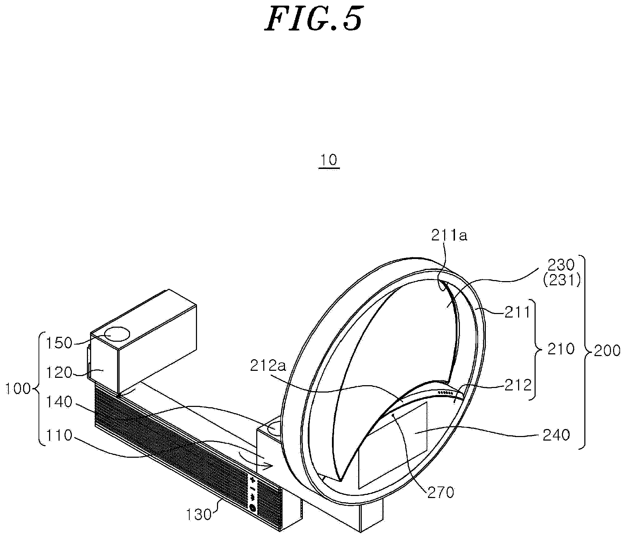

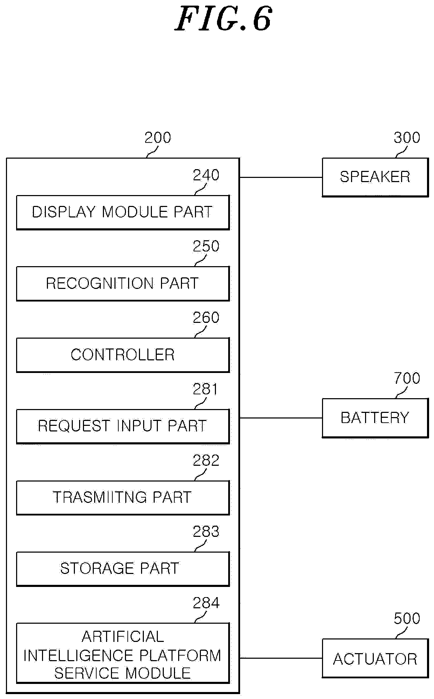

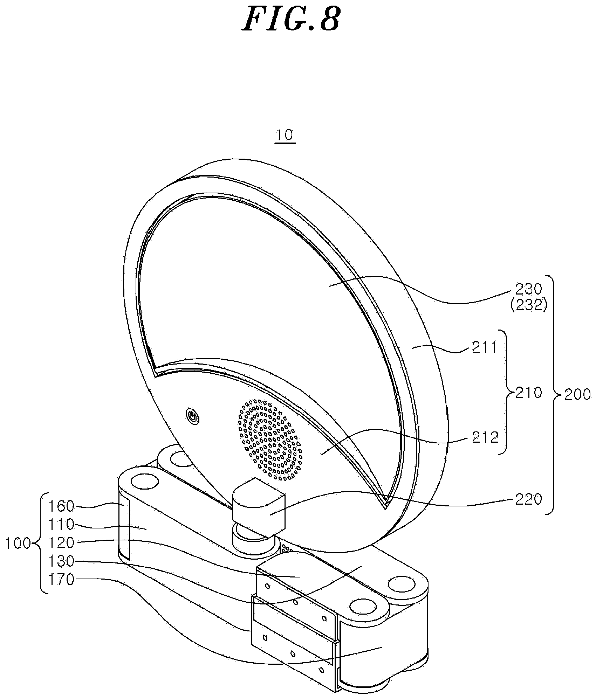

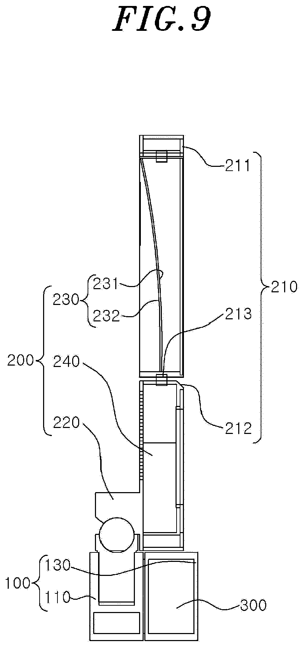

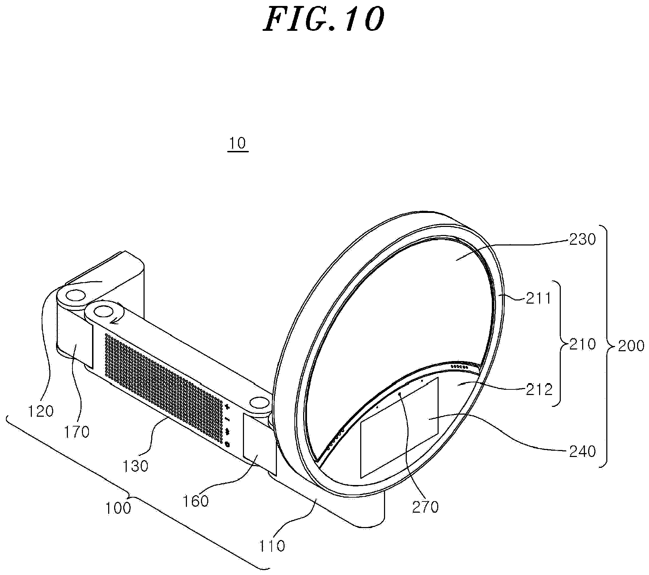

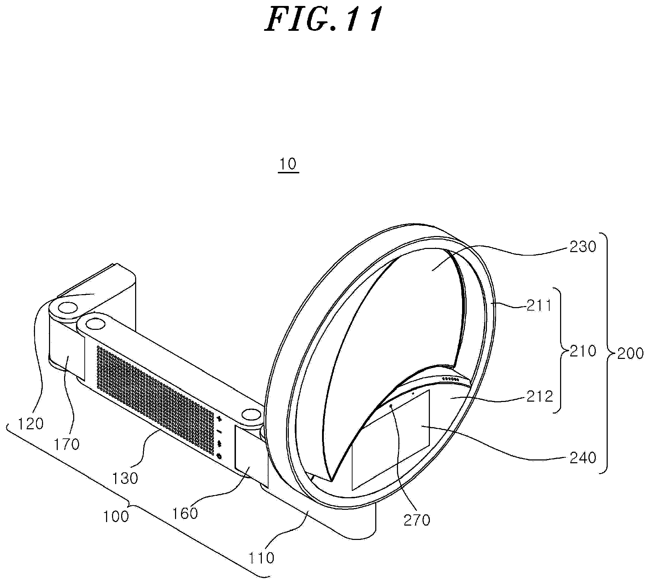

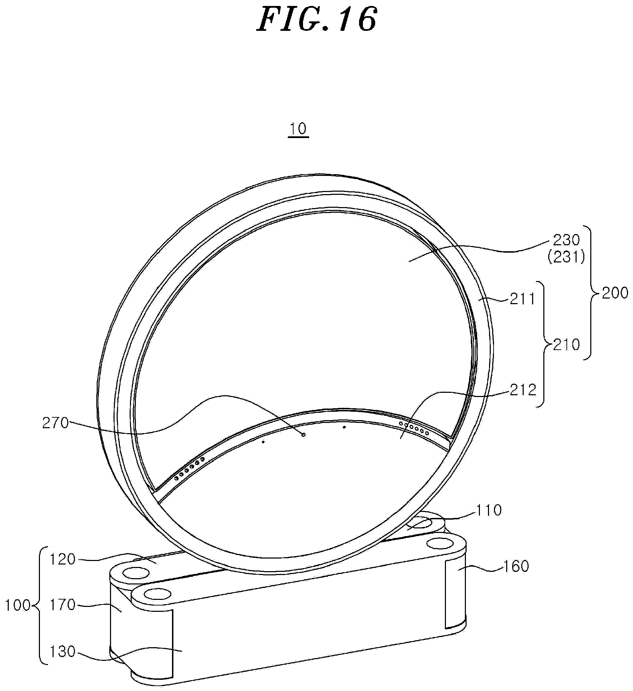

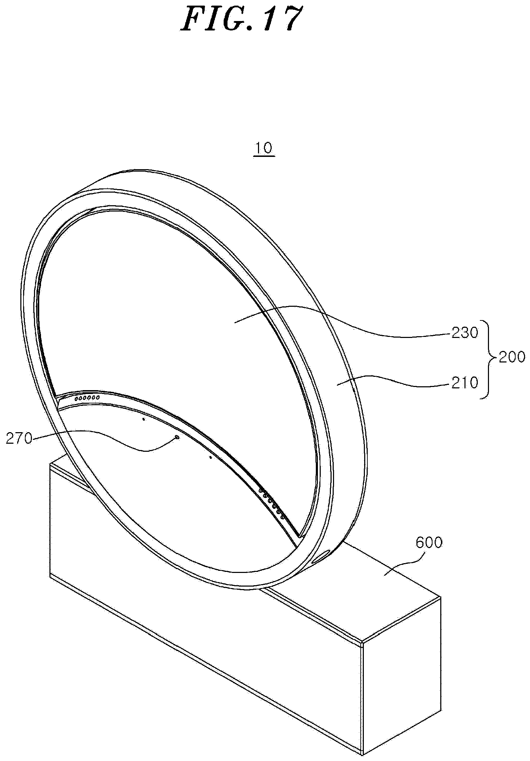

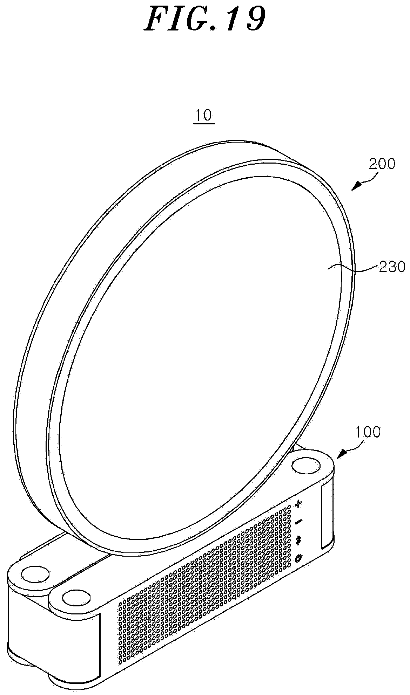

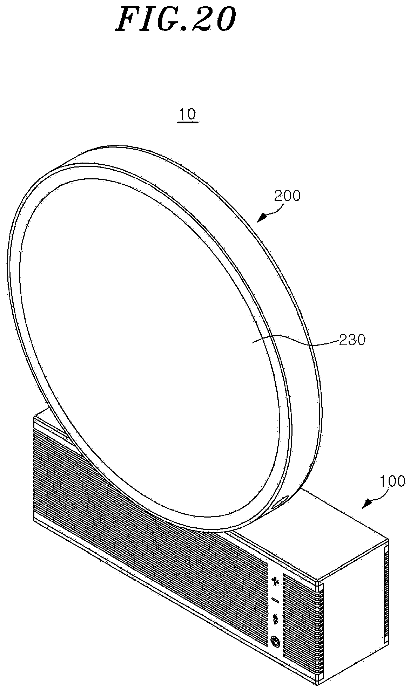

Hereinafter, a specific configuration of a voice recognition based smart mirror device according to embodiments of the present disclosure will be described with reference to FIGS. 1 to 32.

As shown in FIGS. 1 to 6, the voice recognition based smart mirror device 10 according to a first embodiment of the present disclosure may include an arm 100, a mirror unit 200, a speaker 300, a fixing bracket 400, and an actuator 500.

Specifically, the arm 100 may include a plurality of parts rotatably connected to each other in order to adjust various positions of the mirror unit 200. For example, a user can horizontally move the mirror unit 200 to a desired position by adjusting the parts of the arm 100 while a part of the arm 100 is fixed to a wall surface through the fixing bracket 400.

To this end, the arm 100 may include a first arm 110, a second arm 120, a third arm 130, a first rotation shaft 140, and a second rotation shaft 150. The first arm 110 may rotatably connect the mirror unit 200 and the third arm 130. The mirror unit 200 may be rotatably mounted on one end portion of the first arm 110 through a joint part 220, and the third arm 130 may be connected to the other end portion of the first arm 110 through the first rotation shaft 140.

The second arm 110 may be fixedly installed on the wall surface through the fixing bracket 400. The fixing bracket 400 may be mounted on a rear surface of the second arm 110 facing the wall surface, and the second arm 120 may be connected to the third arm 130 through a second rotation shaft 150. The second arm 120 may be positioned on the same horizontal extension line as the first arm 110.

The first arm 110 and the second arm 120 may be rotatably connected to one end portion and the other end portion of the third arm 130 through the first rotation shaft 140 and the second rotation shaft 150, respectively. The third arm 130 may be located below the first arm 110 and the second arm 120.

The speaker 300 may be included in the third arm 130. A plurality of sound slits 131 for outputting the sound of the speaker 300 may be formed in the third arm 130. The sound slit 131 may be provided in the shape of an elongated hole extending in a longitudinal direction of the third arm 130. In addition, the third arm 130 may be provided with various buttons through which the user may increase or decrease the volume of the speaker 300, turn on/off the power of the speaker 300, or execute Bluetooth connection.

The first rotation shaft 140 may connect the other end portion of the first arm 110 and one end portion of the third arm 130 in the up-down direction to allow the first arm 110 and the third arm 130 to relatively rotate. An upper portion of the first rotation shaft 140 may be rotatably fitted into the other end portion of the first arm 110, and a lower portion of the first rotation shaft 140 may be rotatably fitted into one end portion of the third arm 130.

The second rotation shaft 150 may connect one end portion of the second arm 120 to the other end portion of the third arm 130 in the vertical direction to allow the second arm 120 and the third arm 130 to relatively rotate. An upper portion of the second rotation shaft 150 may be rotatably fitted into one end portion of the second arm 120, and a lower portion of the second rotation shaft 150 may be rotatably fitted into the other end portion of the third arm 130.

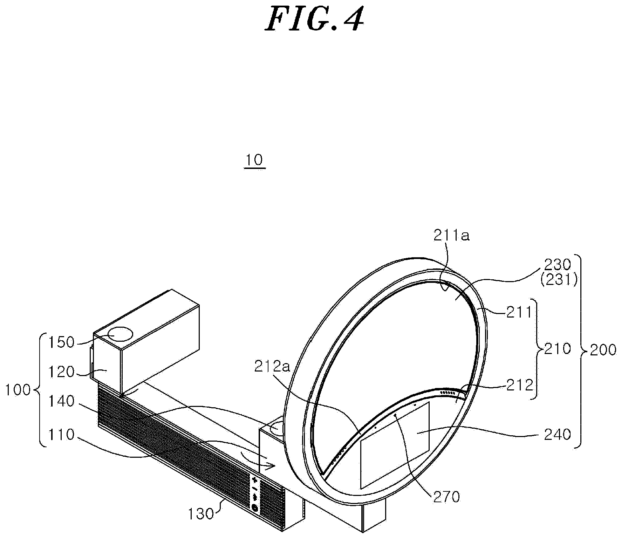

Accordingly, it is possible to rotate the mirror unit 200 at various angles in the range of 180 degrees with respect to the wall surface at which the second arm 120 is fixed, and the distance of the mirror unit 200 from the wall surface can be adjusted by unfolding the first arm 110 and the third arm 130. For example, when the first arm 110 and the third arm 130 are unfolded linearly, a range of the rotation of the mirror unit 200 about the second rotation shaft 150 may be the maximum moving radius of the mirror unit 200 (see FIG. 4).

The mirror unit 200 may provide not only a mirror function, but also a function of diagnosis for skin condition and a healthcare to the user. In addition, the mirror unit 200 may provide music playback, weather information, news information and the like in conjunction with a voice assistant (artificial intelligence secretary platform), and may play music of a smartphone in conjunction with Bluetooth.

Since the mirror unit 200 may be tilted in an up-down direction and in a left-right direction with respect to the arm 100, it may be positioned at various angles with respect to the user's face. In the present embodiment, the mirror unit 200 is configured to have a disk shape as a whole, but the mirror unit 200 may have various shapes without being limited thereto.

The mirror unit 200 may be detachably mounted to the arm 100. The mirror unit 200 may be separated from the arm 100 and then docked to other compatible arms.

The mirror unit 200 may include a body part 210, a joint part 220, a mirror part 230, a display module part 240, a recognition part 250, a controller 260, a detection sensor 270, a request input part 281, a transmitting part 282, a storage part 283, and an artificial intelligence platform service module 284. However, other components may be further included, or some of the components may not be included.

The body part 210 may provide the overall appearance of the mirror unit 200. The body part 210 may be connected to the arm 100 through the joint part 220 such that it may be tilted.

The body part 210 may include a body ring 211, a body support 212, and a body shaft 213. The body ring 211 may have a ring shape and include a through hole 211a in which the mirror part 230 may be mounted. The body support 212 may be provided under the body ring 211.

The body support 212 of the body part 210 may support the display module part 240 under the body ring 211. A curved edge surface 212a may be formed at an upper end portion of the body support 212. The curved edge surface 212a may be formed to be inclined forward to face the user. Further, the curved edge surface 212a may be formed to be curved along the edge so as to be convex upward. Sound holes 212b may be formed in the curved edge surface 212a. The sound holes 212b may output sound of the display module part 240. In particular, since the sound holes 212b are disposed on the curved edge surface 212a formed to be inclined forward, the sound output through the sound holes 212b may be reflected from the lower part of the mirror part 230 to become richer.

In addition, the body shaft 213 may be mounted on the body support 212. The body shaft 213 may connect the mirror part 230 and the body support 212 so that the mirror part 230 may rotate about the body shaft 213 in the through hole 211a.

In addition, the body support 212 may be provided with the recognition unit 250, e.g. a microphone, for detecting a user's voice signal. A plurality of microphones may be provided to be spaced apart from each other at an upper portion in a front surface of the body support 212 so that at least a part of the microphones are exposed. In the present embodiment, there are two microphones, but the present disclosure is not limited thereto, and a different number of microphones may be provided on the body support 212. Preferably, 2 to 8 microphones may be provided.

The joint part 220 may connect the body part 210 and the arm 100 so that the body part 210 can be pivoted in the up-down direction and the left-right direction with respect to the arm 100. The joint part 220 connecting the body part 210 and the arm 100 may have a spherical joint shape, and the body part 210 may rotate smoothly with respect to the arm 100. Since the structure of the joint part 220 corresponds to a conventional rotating structure implemented to enable the pivoting in the up-down and left-right directions, a detailed description thereof will be omitted.

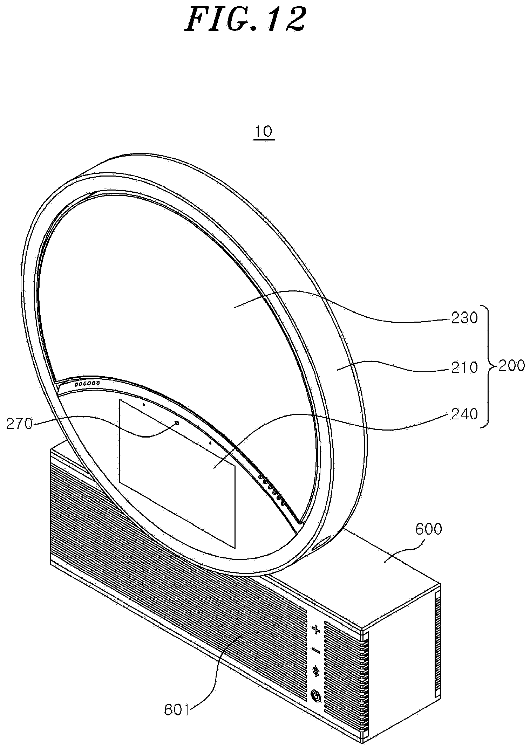

The mirror part 230 may provide a mirror surface that mirrors a user's face. The mirror part 230 may rotate in the left-right direction about the center of the body part 210 with respect to an axis extending perpendicularly from the ground (see FIG. 12). The axis extending perpendicularly from the ground may be the axis of the body shaft 213 installed at the center of the body part 210.

The mirror part 230 may include a convex mirror part 231 convexly formed toward the front of the mirror unit 200 and a concave mirror part 232 formed concavely toward the rear of the mirror unit 200. The concave mirror part 232 may be a magnifying glass that enlarges and reflects the user's face.

The display module part 240 may be mounted on the body support 212 of the body part 210 to be exposed to the front side of the body part 210 to output an image. The display module part 240 may include a terminal that provides a display in the form of a touch pad capable of sensing a user's touch signal. The display module part 240 may output an image by executing a preset display mode (application, programming, etc.) according to a user's voice signal or touch signal.

In this case, the display mode may include a voice assistant mode. The voice assistant mode may provide various information according to a preset algorithm, e.g., an algorithm to be implemented in a voice assistant of a conventional display module part, or an algorithm for receiving corresponding information from a server, in response to a user's voice signal or touch signal.

The information provided by such an algorithm may be, for example, daily information such as weather, information on people and objects, or the like, but the spirit of the present disclosure is not necessarily limited thereto. When a user's voice signal is received in the voice assistant mode, weather information, through the preset algorithm, news information, music information, security information, beauty content information, healthcare information, latest trend information, makeup information, hair and nail-related information, and the like may be provided as voice through the speaker 300 or as a screen of the display module part 240.

In addition, the voice assistant of the display module part 240 may control the operation of peripheral electronic products using a conventional smart home or loT technology. For example, if a user provides a voice signal to operate peripheral products (light, bathroom toilet, shower, bath temperature, amount of water, etc.) to the voice recognition based smart mirror device according to the disclosure, the voice assistant may control the peripheral products through a voice command using smart home or loT technology.

For example, if a user provides a voice signal "Drain water from a toilet" to the voice recognition based smart mirror device according to the present disclosure saying, the voice assistant may use smart home or loT technology to discharge the water contained in the toilet to the outside. In addition, if the user provides voice signals such as "Set the water temperature in the shower to 39 degrees Celsius," "Set the water temperature in the bath to 43 degrees Celsius," "Turn on the bathroom light," "Turn off the bathroom light," or "Change the bathroom light to blue," the voice assistant can control the lighting, and water temperature or volume of a shower.

The recognition unit 250 may include a component capable of recognizing a user's voice signal. For example, the recognition unit 250 may include a microphone through which a user's voice signal may be input. The microphone may have a water proof function and a dust resistant function.

The controller 260 may control the display module part 240 according to a touch signal sensed through the display of the display module part 240. In addition, the controller 260 may control the display module part 240 by executing a voice assistant function by a voice signal sensed by the microphone of the recognition unit 250. Here, the voice assistant function may be implemented as a voice assistant (artificial intelligence secretary platform) to which voice recognition technology is applied to convert voice signals received through microphones into words or sentences.

Examples of such voice assistants include Amazon's "Alexa," Google's "Google Assistant," SK Telecom's "NUGU," and Naver's "Clova." The voice assistant function processes the user's voice signal as an input signal and provides various services, as an artificial intelligence assistant service, such as information search, music playback, schedule management, alarm service reservation guidance function operation of the speaker 300.

In addition, the controller 260 may move (rotate) the mirror unit 200 in close contact with the wall to a specific position by controlling the operation of the actuator 500 using the voice assistant function.

For example, if the user provides a voice signal to the recognition unit 250 to move the mirror unit 200 in close contact with the wall to a specific position, the voice assistant may apply an operation signal to the actuator 500 to move the mirror unit 200 to the specific position. When the operation signal is applied to the actuator 500, first, the actuator 500 rotates the second rotation shaft 150 by a preset angle so that the third arm 130 rotates to a preset position. In this case, the actuator 500 may rotate the joint part 220 of the mirror unit 200 so that the mirror unit 200 maintains parallel to the wall surface. In addition, when the second rotation shaft 150 is rotated by a preset angle, the actuator 500 rotates the first rotation shaft 140 by a preset angle so that the first arm 110 is positioned to a preset position. In this case, the actuator 500 may rotate the joint part 220 of the mirror unit 200 so that the mirror unit 200 maintains parallel to the wall surface. When the first rotation shaft 140 is rotated by a preset angle, the actuator 500 rotates the joint part 220 by a preset angle so that the mirror unit 200 is rotated to a preset position.

For example, if the user provides a voice signal to the recognition unit 250 to move the mirror unit 200 in close contact with the wall to a specific position, the voice assistant may apply an operation signal to the actuator 500 to move the mirror unit 200 to the specific position. When the operation signal is applied to the actuator 500, first, the actuator 500 rotates the second rotation shaft 150 by a preset angle so that the third arm 130 rotates to a preset position. In this case, the actuator 500 may rotate the joint part 220 of the mirror unit 200 so that the mirror unit 200 maintains parallel to the wall surface. In addition, when the second rotation shaft 150 is rotated by a preset angle, the actuator 500 rotates the first rotation shaft 140 by the preset angle so that the first arm 110 is rotated to a preset position. In this case, the actuator 500 may rotate the joint part 220 of the mirror unit 200 so that the mirror unit 200 maintains parallel to the wall surface. When the first rotation shaft 140 is rotated by a preset angle, the actuator 500 rotates the joint part 220 by a preset angle so that the mirror unit 200 is rotated to a preset position.

In addition, the controller 260 may move (rotate) the mirror unit 200 deployed to the specific position to an initial position where the mirror unit 200 is in close contact with the wall by controlling the operation of the actuator 500 using the voice assistant function.

For example, if the user provides a voice signal to the recognition unit 250 to move the mirror unit 200 deployed to the specific location to the initial location where the mirror unit 200 is in close contact with the wall, the voice assistant may apply an operation signal for moving the mirror unit 200 to the initial position to the actuator 500. When the operation signal is applied to the actuator 500, first, the actuator 500 rotates the second rotation shaft 150 such that the third arm 130 is positioned to cross an angle perpendicular to the wall surface. When the third arm 130 is positioned to cross the angle perpendicular to the wall surface, the actuator 500 rotates the joint part 220 so that the mirror unit 200 is positioned on the same plane as the first arm 110. When the mirror unit 200 is positioned on the same plane as the first arm 110, the actuator 500 rotates the first rotation shaft 140 so that the first arm 110 is positioned to overlap the third arm 130 in the up-down direction. When the first arm 110 is positioned to overlap the third arm 130 in the up-down direction, the actuator 500 rotates the second rotation shaft 150 such that the first arm 110, the third arm 130 and the mirror unit 200 are positioned in their initial positions.

The detection sensor 270 may be provided between a pair of microphones. The detection sensor 270 may be a type of sensor capable of detecting a user. For example, when a user (e.g., a hand of the user) approaches the detection sensor 270 within a predetermined distance, the detection sensor 270 may detect the user who is positioned within the predetermined distance. When the detection sensor 270 detects the approach of the user, the controller 260 may control the operation of the mirror unit 200 according to a preset program. For example, even if the user does not directly touch the mirror unit 200, the operation of the mirror unit 200 may be implemented by the detection of the detection sensor 270.

For example, when the approach of the user is continuously detected for a certain period of time after the initial detection by the detection sensor 270, the controller 260 may turn on the power of the mirror unit 200. When the approach of the user is repeatedly detected by the detection sensor 270 for a preset short period of time, the controller 260 may operate the mood lamp installed on the edge of the mirror unit 200 and may change the color of the mood lamp (e.g., LED lamp) into, e.g., one of cool, warm, and white colors. When the power of the mirror unit 200 is turned on and the user's approach is continuously detected for a predetermined time by the detection sensor 270, the controller 260 may turn off the power of the mirror unit 200. Further, the present disclosure is not limited thereto, and the controller 260 may variously change the operation of the mirror unit 200 based on the duration and frequency of the detection for the user by the detection sensor 270.

The request input part 281 is configured to receive an input from a user. The request input part 281 may be implemented by a configuration such as a microphone.

The transmitting part 282 is a module for performing communication, and may be implemented by a wired or wireless communication module. Through the transmitting part 282, the mirror unit 200 may transmit a predetermined signal to the biometric information measuring device or receive a predetermined signal from the biometric information measuring device.

Here, the biometric information measuring device may determine the user's biometric information based on at least one bio-signals. For example, the biometric information measuring device may determine a photoplethysmography, an electrocardiogram, a heart rate, a heart rate variability, a blood pressure, and/or oxygen saturation (e.g., peripheral oxygen saturation) of a user based on the at least one bio-signals, e.g., a PPG signal and/or an ECG signal. The biometric information measuring device may provide the mirror unit 200 with the result of the operation performed by the biometric information measuring device. The biometric information measuring device may be attached to and detached from the mirror unit 200. For detaching and attaching the biometric information measuring device, a hook and a clasp or a magnet may be used.

The storage part 283 is a means for storing data, and may be implemented by various means such as a memory. The storage part 283 may store a criterion indicating that the biometric information is normal. The criteria may include numerical reference ranges. For example, the criterion for each of the biometric information may be stored in the form of a numerical reference range. The reference range may be a range of numerical values that may indicate that the user's biometric information is normal, that is, there is no abnormality in the user's health.

The artificial intelligence platform service module 284 may provide artificial intelligence services. The artificial intelligence platform service module 284 may perform various functions. For example, the artificial intelligence platform service module 284 may recognize a voice signal according to a user's utterance and communicate with the user in real time.

In addition, the artificial intelligence platform service module 284 may provide various additional services. For example, a specific operation may be performed with an external device electrically connected to the mirror unit 200 based on a recognized voice signal for the user's utterance. The specific operation may include the provision of the user's biometric information measured daily, weekly, and monthly, and the analysis and evaluation of the user's biometric information, the provision of information on an alarm and alert (notification) function, the provision of information on schedule, the provision of a specific image service, Internet search, online shopping, and the like.

The artificial intelligence platform service module 284 may be implemented by at least one component, e.g., a hardware component or a software component.

When the signal including the biometric information is received from the biometric information measuring device through the transmitting part 282, the artificial intelligence platform service module 284 determines whether the biometric information deviates from a preset criteria indicating a normal range of the biometric information by comparing the received biometric information with a reference stored in the storage part 283. As a result of the comparison, when it is confirmed that the received biometric information deviates from the predetermined criteria, the artificial intelligence platform service module 284 provides a notification to the server and the mobile application or the like which are connected to the mirror unit 200 in communication.

Further, when receiving a signal including biometric information, the artificial intelligence platform service module 284 may compare the received biometric information with the biometric information pre-stored in the storage part 283. When the difference between the pre-stored biometric information and the received biometric information exceeds a reference range stored in the storage part 283, the artificial intelligence platform service module 284 may identify or determine the received biometric information as deviating from the criteria.

Further, when it is determined that the biometric information deviates from the preset criteria stored in the storage part 283, the artificial intelligence platform service module 284 may control the first light emitting diode provided in the mirror unit 200 to emit light. The artificial intelligence platform service module 284 may control the second light emitting diode provided in the mirror unit 200 to emit light when it is determined that the biometric information does not deviate from the preset criteria.

In addition, when the mirror unit 200 receives biometric information from the biometric information measuring device through the transmitting part 282, the artificial intelligence platform service module 284 causes the third light emitting diode provided in the mirror unit 200 to emit light. Accordingly, the user may be provided with a notification indicating that the biometric information has been received through the artificial intelligence platform service module 284 and the mobile application.

The mirror unit 200 may not provide a precise method of prescribing telemedicine for certain diseases, but a non-contact remote monitoring function which determines whether basic biometric information of the user who has the mirror unit 200 and the biometric information measuring device, e.g., body fat, body water, a stress index, a photoplethysmography, an electrocardiogram, a heart rate, a heart rate variability, a blood pressure, and/or oxygen saturation (e.g., peripheral oxygen) is out of a predetermined reference range (standard range).

When it is determined that the biometric information is out of a predetermined reference range, the healthcare service providing device may provide a designated notification through a display, a speaker, or a mobile application. The designated notification may include a basic guide providing a user with a guide to visit the hospital immediately or within a few days or basic simple prescription and health care information. Alternatively, the mirror unit 200 may transmit the biometric information to a specific server, and the specific server determines whether the biometric information is out of a predetermined reference range. And then, when the specific server determines that the biometric information is out of a predetermined reference range, the notification is transmitted to the mirror unit 200 so that the user of the mirror unit 200 may check the notification.

For example, in a country where the supply rate of medical facilities is low, medical benefits are low, and medical costs are high, the electronic device including the mirror unit 200 and the biometric information measuring device according to the embodiment of the present disclosure may be useful. When the electronic device including the mirror unit 200 and the biometric information measuring device according to the embodiment of the present disclosure is provided to a user who is burdened with hospital costs or does not easily visit a hospital due to transportation, or the like, the electronic device may provide a simple prescription such as aspirin or inform the user of an urgent crisis that requires a quick visit to the hospital. In other words, the electronic device including the mirror unit 200 and the biometric information measuring device according to the embodiment of the present disclosure may be used as a device for implementing an auxiliary function of telemedicine.

Various embodiments of the present disclosure may be implemented as software (e.g., program) including instructions stored in machine (e.g., computer)-readable storage media (e.g., memory (internal memory or external memory). The machine is a device that invokes the stored instructions from the storage media and is operable in accordance with the invoked instructions, and may include an electronic device (e.g., electronic device) according to the disclosed embodiments. When the command is executed by a processor (e.g., processor), the processor may perform functions corresponding to the instructions, either directly or by using other components under controller of the processor. The instructions may include code generated by a compiler or executable by an interpreter. The machine-readable storage media may be provided in the form of non-transitory storage media. Here, the term 'non-transitory' means that the storage media do not include a signal and is tangible, but do not distinguish whether data is stored semi-permanently or temporarily in the storage media.

The speaker 300 may output a voice signal of the voice assistant. For example, the speaker 300 may be controlled by a voice assistant of the controller 260. The speaker 300 may be embedded in the third arm 130 to output sound from the arm 100. The speaker 300 may communicate with the controller 260 in a Bluetooth communication method.

The fixing bracket 400 may fix the arm 100 to the wall surface. The fixing bracket 400 may be fixed to the wall surface as well as the second arm 120. In order to fix the fixing bracket 400 to the wall surface, a plurality of fixing bolts (screws) may be used.

The actuator 500 may include a servo motor for rotating the arm 100. The servo motor may include a plurality of servo motors that may be respectively connected to the first rotation shaft 140, the second rotation shaft 150, and the joint part 220. The servo motors connected to the first rotation shaft 140, the second rotation shaft 150, and the joint part 220 may independently rotate the third arm 130 connected to the second arm 120, the first arm 110 connected to the third arm 130, and the mirror unit 200 connected to the joint part 220 under the control of the controller 260. The servo motors may be controlled by an operation signal of the controller 260.

For example, the actuator 500 may be linked to the voice assistant of the controller 260 so that the position of the mirror unit 200 is controlled by the user's voice signal. By shifting the position of the mirror unit 200 under the control of the controller 260, the actuator 500 may allow the mirror unit 200 to be directed to the user's face at various angles.

A battery 700 may be embedded in the mirror unit 200. The battery 700 may be charged by receiving power from the outside through a charging port. The battery 700 may supply the charged power to the mirror unit 200, the speaker 300, the actuator 500, and the like.

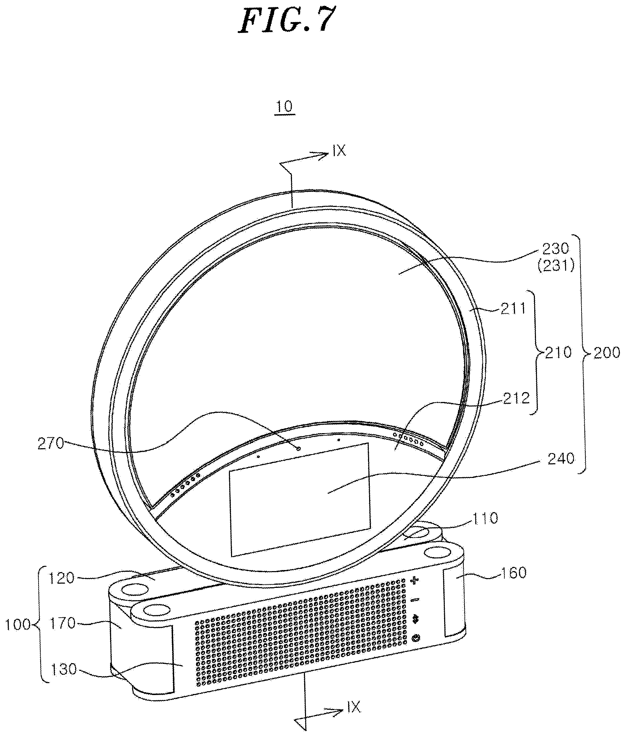

As shown in FIGS. 7 to 11, according to the second embodiment of the present disclosure, the arm 100 of the voice recognition based smart mirror device 10 may include a plurality of parts rotatably linked to each other to adjust various positions of the mirror unit 200.

In describing the second embodiment of the present disclosure, the second embodiment is different from the first embodiments described above in that the arm 100 of the second embodiment includes the first arm 110, the second arm 120, the third arm 130, a first link 160, and a second link 170, and the same configurations as those in the first embodiment are given the same reference numerals and the descriptions in the first embodiments are referred.

The first arm 110 may rotatably connect the mirror unit 200 and the third arm 130. The mirror unit 200 may be rotatably mounted to one end portion of the first arm 110 through the joint part 220, and the third arm 130 may be linked to the other end portion of the first arm 110 through the first link 160.

The second arm 120 may be fixedly installed on the wall surface through the fixing bracket 400. The fixing bracket 400 may be mounted on a rear surface of the second arm 120 facing the wall surface, and one end portion of the second arm 120 may be connected to the third arm 130 through the second link 170. The second arm 120 may be positioned on the same horizontal extension line as the first arm 110.

The first arm 110 and the second arm 120 may be rotatably connected to one end portion and the other end portion of the third arm 130 through the first link 160 and the second link 170, respectively. The speaker 300 may be embedded in the third arm 130. The sound slits 131 for outputting the sound of the speaker 300 may be formed in the third arm 130. The third arm 130 may be positioned on the same horizontal extension line as the first arm 110 and the second arm 120.

The first link 160 may link one end portion of the first arm 110 and one end portion of the third arm 130 in a horizontal direction so that the first arm 110 and the third arm 130 are rotatably connected to each other. One end portion of the first link 160 may be rotatably connected to the other end portion of the first arm 110, and the other end portion of the first link 160 may be rotatably connected to one end portion of the third arm 130.

The second link 170 may link one end portion of the second arm 120 to the other end portion of the third arm 130 in a horizontal direction so that the second arm 120 and the third arm 130 rotate with each other. One end portion of the second link 170 may be rotatably connected to one end portion of the second arm 120, and the other end portion of the second link 170 may be rotatably connected to the other end portion of the third arm 130.

Accordingly, it is possible to rotate the mirror unit 200 at various angles in the range of 180 degrees with respect to the wall surface at which the second arm 120 is fixed, and the distance of the mirror unit 200 from the wall surface can be adjusted by unfolding the first arm 110 and the third arm 130, which is farther than the distance of the mirror unit 200 from the wall surface in the first embodiment.

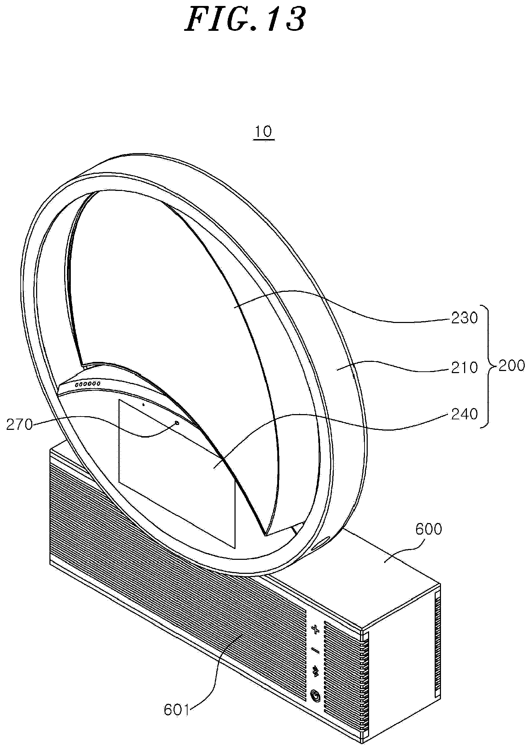

As shown in FIGS. 12 and 13, the voice recognition based smart mirror device 10 according to the third embodiment of the present disclosure may include a main body 600, a mirror unit 200, and a speaker 300.

In describing the third embodiment of this disclosure, the third embodiment is different from the first and second embodiments described above in that the voice recognition based smart mirror device 10 includes the main body 600 instead of the arm. This difference will be mainly described, and the same configurations as those in the first and second embodiments are given the same reference numerals and the descriptions in the first and second embodiments are referred.

The main body 600 may rotatably support the mirror unit 200. The main body 600 may be provided in the form of a hexahedral box. A battery 700 may be provided inside the main body 600, and main body slits 601 for outputting sound of the speaker 300 may be formed on the front and rear circumferential surfaces of the main body 600. The main body slit 601 may have an elongated hole extending in a longitudinal direction of the main body 600. In this case, the speaker 300 may communicate with the controller 260 of the mirror unit 200 in a Bluetooth communication method.

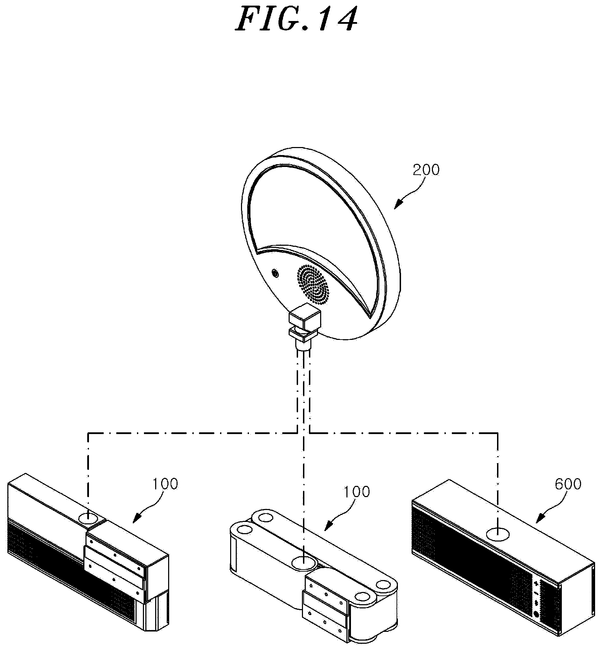

As shown in FIG. 14, the mirror unit 200 may be separated from the arm 100, and the separated mirror unit 200 may be docked to different compatible arms 100. For example, the arm 100 separated from the arm 100 of the first embodiment may be selectively docked to the arm 100 of the second embodiment or the main body 600 of the third embodiment.

In particular, when the mirror unit 200 is separated from the arm 100 or the main body 600 or docked to the arm 100 or the main body 600 while the sound is being output, the controller 260 may control the display module part 240 and the speaker 300 to maintain a continuous output of sound.

For example, when the mirror unit 200 is separated from the arm 100 or the main body 600 while the sound is being output through the sound slits 131 of the arm 100 or the main body slits 601 of the main body 600, the output of the sound through the sound slits 131 of the arm 100 or the main body slits 601 of the main body 600 may be stopped, and simultaneously, the sound through the sound holes 212b of the mirror unit 200 may be implemented. Accordingly, when the mirror unit 200 is separated from the arm 100 or the main body 600, the sound can be output through the sound holes 212b of the mirror unit 200, which prevents sound disconnection in advance.

In addition, when the mirror unit 200 is docked in the arm 100 or the main body 600 while the sound is being output through the sound holes 212b of the mirror unit 200, the output of sound through the sound holes 212b of the mirror unit 200 may be stopped, and simultaneously, output of sound through the sound slits 131 of the arm 100 or the main body slits 601 of the main body 600 may be implemented. Accordingly, when the mirror unit 200 is docked to the arm 100 or the main body 600, the sound can be output through the sound slits 131 of the arm 100 or the main body slits 601 of the main body 600, which prevents sound disconnection in advance.

Meanwhile, in the present embodiment, the mirror unit 200 provided with the display module part 240 and the arm 100 on which the speaker 300 is mounted have been described, however, the present disclosure is not limited thereto, and the display module part 240 may be selectively installed in the mirror unit 200 and the speaker 300 may be selectively installed in the arm 100.

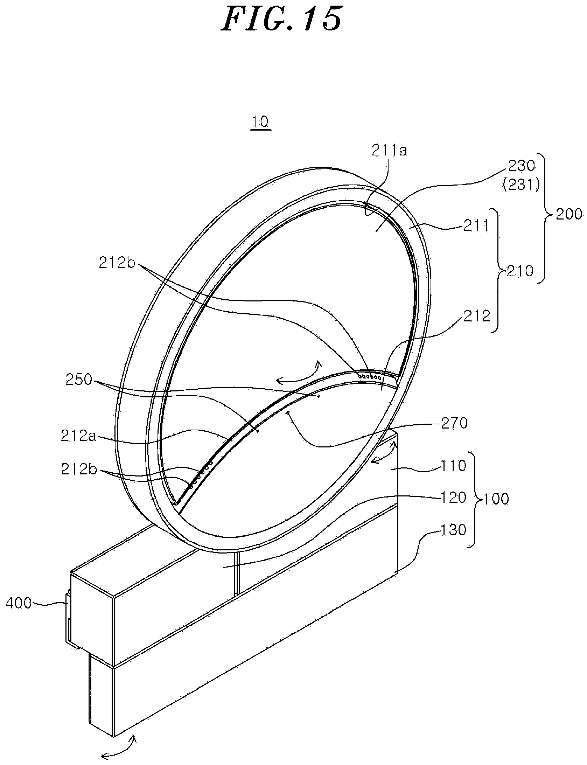

For example, as shown in FIGS. 15 to 17, the display module part 240 may not be installed in the mirror unit 200, and the speaker 300 may not be installed in the arm 100.

In addition, in the present embodiment, the shape of the mirror part 230 is formed in a crescent shape, but it is not limited thereto, and the shape of the mirror part 230 may be provided in various shapes other than the crescent shape.

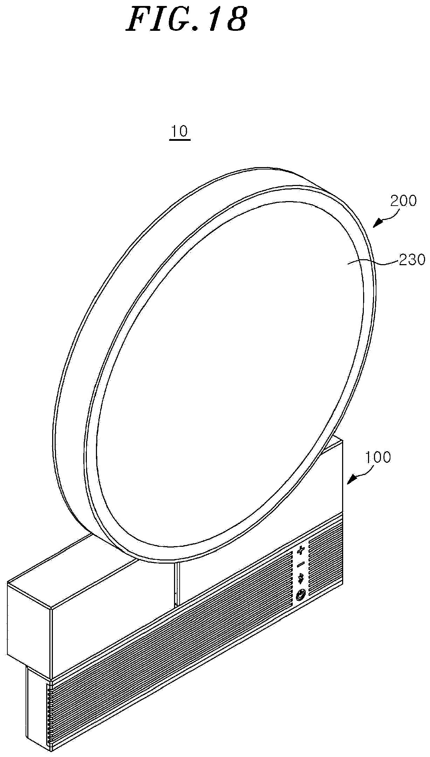

For example, as shown in FIGS. 18 to 20, the mirror part 230 may have a circular shape or a full moon shape.

In addition, in the present embodiment, the arm of the voice recognition based smart mirror device 10 is connected to the mirror unit 200 to rotate in the width direction has been described, but the arm of the voice recognition based smart mirror device 10 may be connected the mirror unit 200 to rotate in the up and down direction.

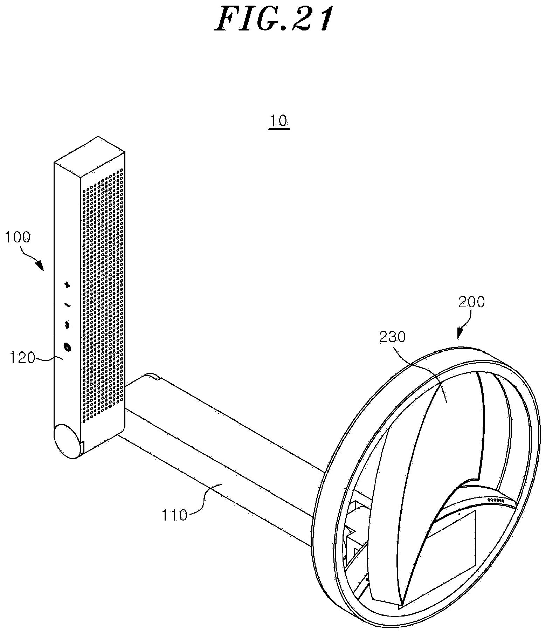

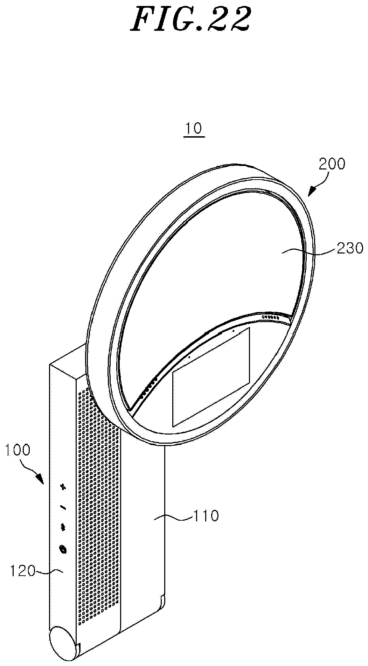

For example, as shown in FIGS. 21 and 22, the arm 100 of the voice recognition based smart mirror device 10 may include a first arm 110 and a second arm 120 connected to the mirror unit 200 in the up and down direction. One end of the first arm 110 may be connected to the mirror unit 200 to rotate in the up and down direction, and the other end of the first arm 110 may be connected to the end portion of the second arm 120 to rotate in the up and down direction. When the first arm 110 is rotated at an end of the second arm 120 and folded into a wall surface (wall), the second arm 120 may be positioned on the same plane as the first arm 110.

The second arm 120 may be connected to the first arm 110 to rotate in the up and down direction. The rear surface of the second arm 120 may be fixedly installed on the wall surface. A speaker may be embedded in the second arm 120. A sound hole for outputting sound from a speaker may be formed in the front surface of the second arm 120.

In addition, the shape of the mirror part 230 of the mirror unit 200 may be formed in a crescent shape. Further, the shape of the mirror part 230 may also be provided in various shapes other than the crescent shape.

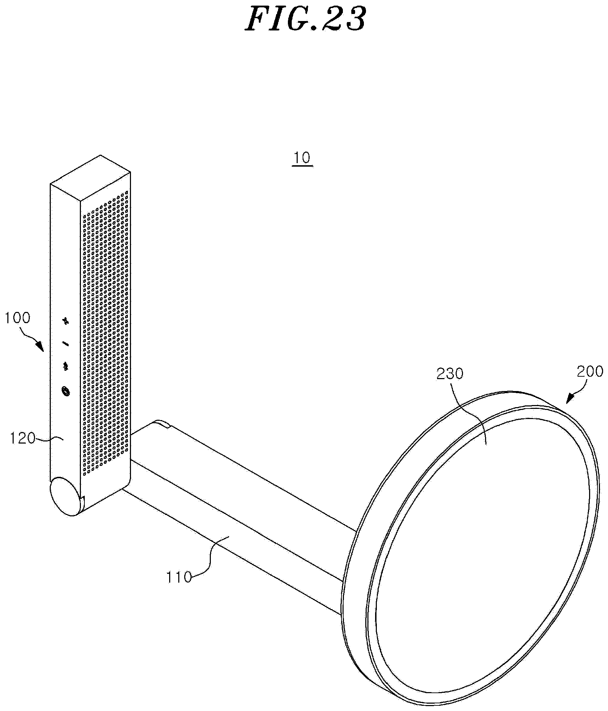

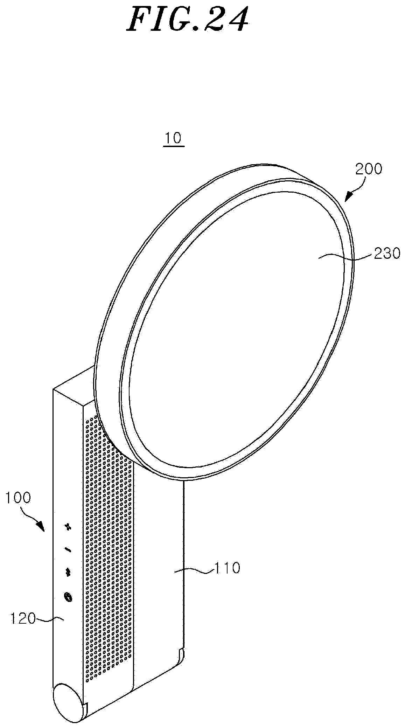

As shown in FIGS. 23 and 24, the mirror part 230 may have a circular shape (a full moon shape). Other than the shape of the mirror part 230, the rest of the configuration corresponds to the configuration described above (see, FIGS. 21 to 22), so a detailed description thereof will be omitted.

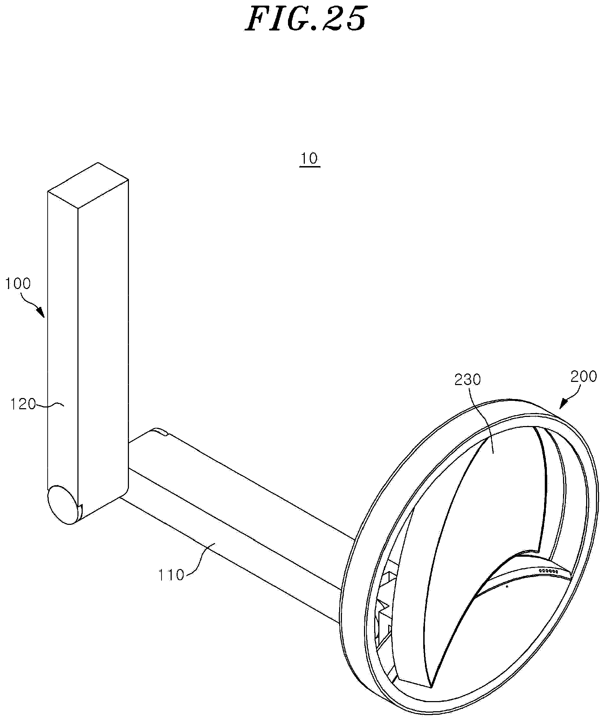

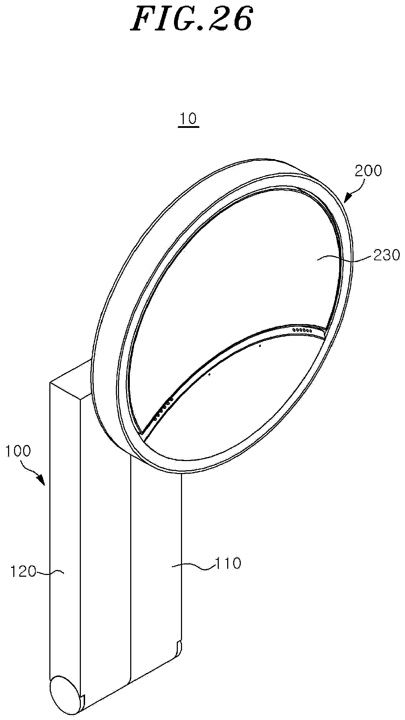

As shown in FIGS. 25 and 26, the arm 100 of the voice recognition based smart mirror device 10 may include a first arm 110 and a second arm 120 connected to the mirror unit 200 in the up and down direction. One end of the first arm 110 may be connected to the mirror unit 200 to rotate in the up and down direction, and the other end of the first arm 110 may be connected to the end portion of the second arm 120 to rotate in the up and down direction.

The second arm 120 may be connected to the first arm 110 to rotate in the up and down direction. A rear surface of the second arm 120 may be fixedly installed on a wall surface. When the first arm 110 is rotated at the end portion of the second arm 120 and folded against the wall surface, the second arm 120 may be positioned on the same plane as the first arm 110.

In addition, the shape of the mirror part 230 of the mirror unit 200 may be formed in a crescent shape. Further, the shape of the mirror part 230 may also be provided in various shapes other than the crescent shape.

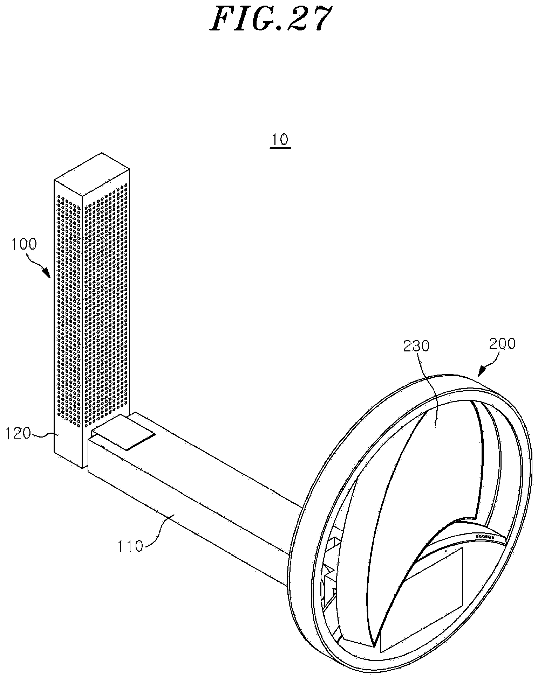

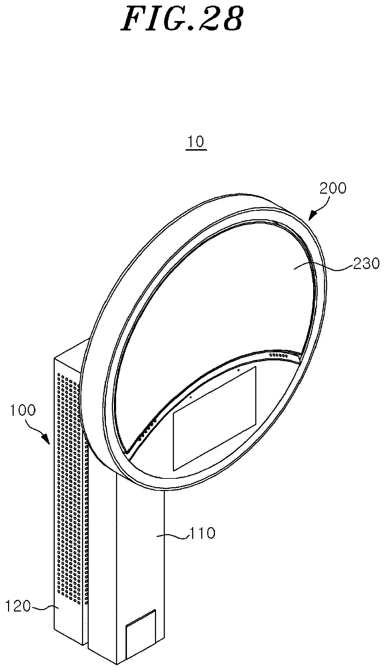

As shown in FIGS. 27 and 28, the mirror part 230 may have a circular shape (a full moon shape). Other than the shape of the mirror part 230, the rest of the configuration corresponds to the configuration described above (see, FIGS. 25 and 26), so a detailed description thereof will be omitted.

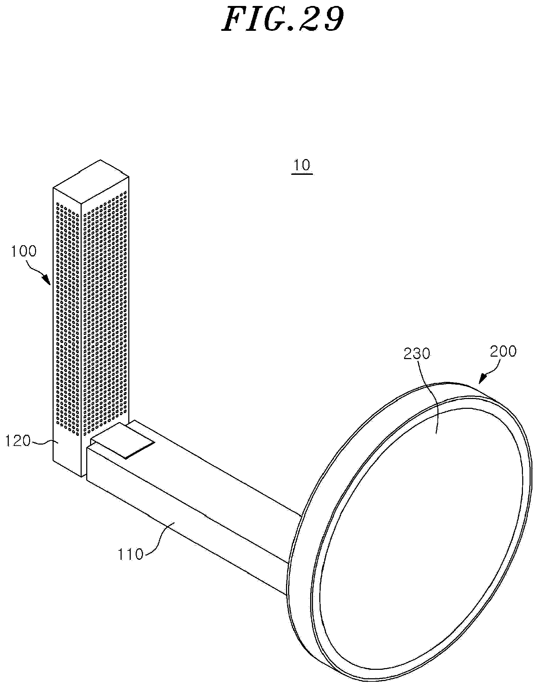

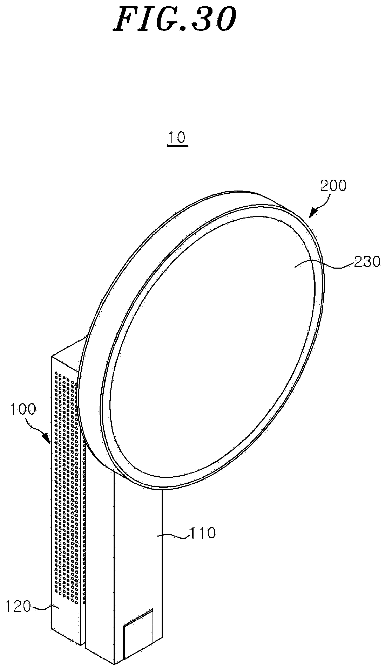

As shown in FIGS. 29 and 30, the arm 100 of the voice recognition based smart mirror device 10 may include a first arm 110 and a second arm 120 connected to the mirror unit 200 in the up and down direction. One end of the first arm 110 may be connected to the mirror unit 200 to rotate in the up and down direction, and the other end of the first arm 110 may be connected to the end portion of the second arm 120 to rotate in the up and down direction.

The second arm 120 may be connected to the first arm 110 to rotate in the up and down direction. A rear surface of the second arm 120 may be fixedly installed on a wall surface. When the first arm 110 is rotated at the end of the second arm 120 and folded against the wall, the second arm 120 may be positioned to overlap the front side of the first arm 110. A speaker may be embedded in the second arm 120. Sound holes for outputting sound from a speaker may be formed in the side and front surfaces of the second arm 120.

In addition, the shape of the mirror part 230 of the mirror unit 200 may be formed in a crescent shape. Further, the shape of the mirror part 230 may also be provided in various shapes other than the crescent shape.

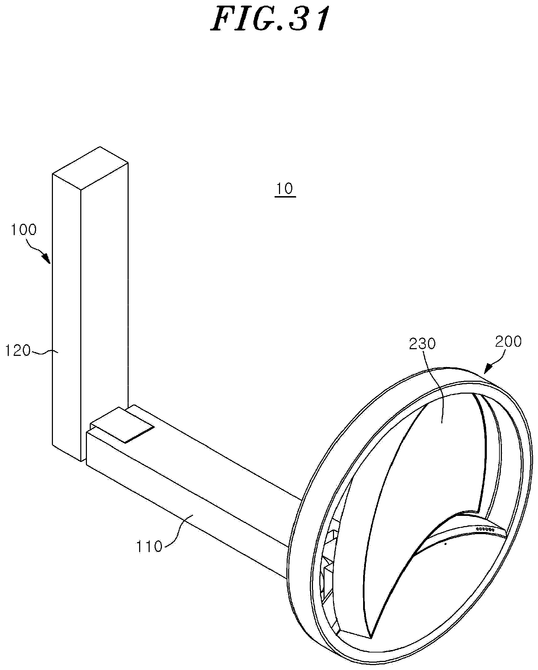

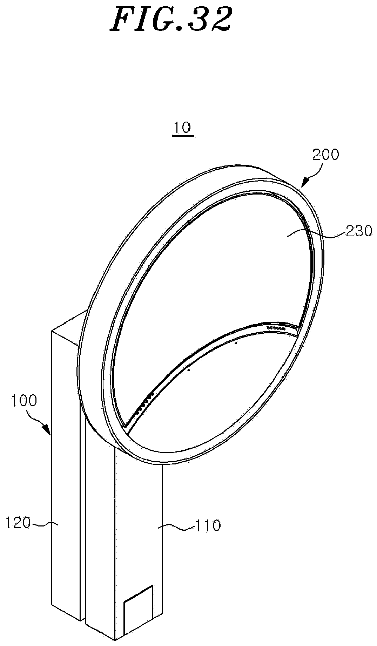

As shown in FIGS. 31 and 32, the mirror part 230 may have a circular shape (a full moon shape). Other than the shape of the mirror part 230, the rest of the configuration corresponds to the configuration described above (see, FIGS. 29 and 30), so a detailed description thereof will be omitted.

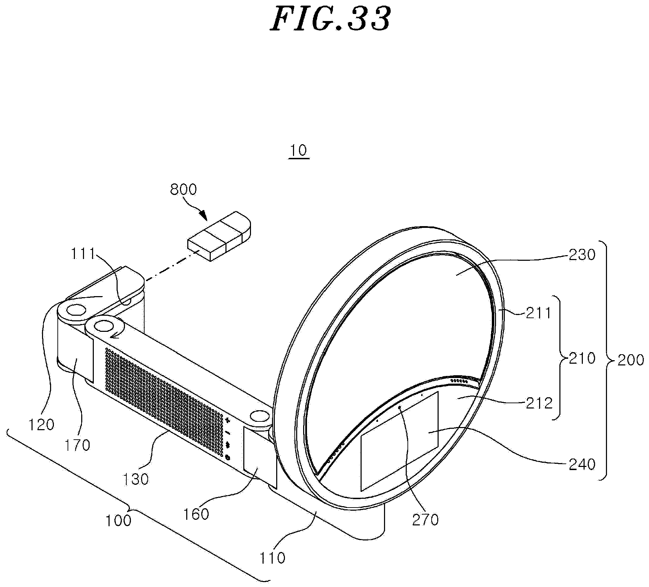

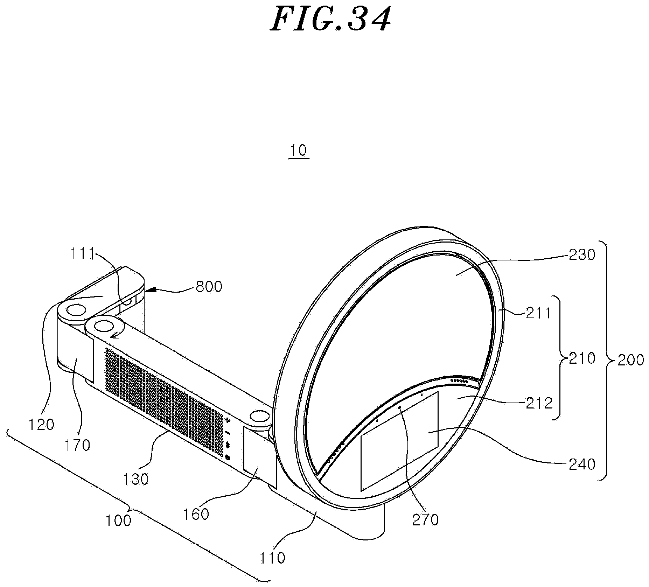

As shown in FIGS. 33 and 34, the second arm 120 may include a detachable portion 111 in which the biometric information measuring device 800 is mounted. The detachable portion 111 may include a seating space for attaching the biometric information measuring device 800 to the voice recognition based smart mirror device 10. When the biometric information measuring device 800 is seated on the detachable portion 111 of the second arm 120, the biometric information measuring device 800 may measure at least one biometric information of the user.

For example, when the biometric information measuring device 800 is mounted in the detachable portion 111 of the second arm 120, the biometric information measuring device 800 may measure a body fat, body water, a stress index, a photoplethysmography, an electrocardiogram, a heart rate, a heart rate variability, a blood pressure, and/or oxygen saturation (e.g., peripheral oxygen saturation) of the user. Further, the biometric information measuring device 800 may provide the biometric information measured by itself to the mirror unit 200 through a wired or wireless communication module. The mirror unit 200 may transmit a predetermined signal to the biometric information measuring device 800 or receive a predetermined signal from the biometric information measuring device 800.

Although FIG. 33 shows that the biometric information measuring device 800 is accommodated in the second arm 120, the present disclosure is not limited thereto, the biometric information measuring device 800 may be accommodated in another part of the arm 100 or the mirror unit 200. In addition, the biometric information measuring device 800 may be coupled to the arm 100 or the mirror unit 200 through a latch or a magnet rather than a seating groove.

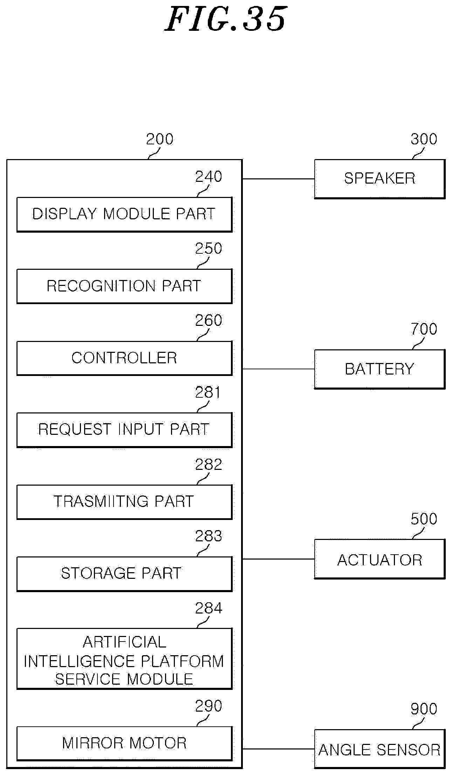

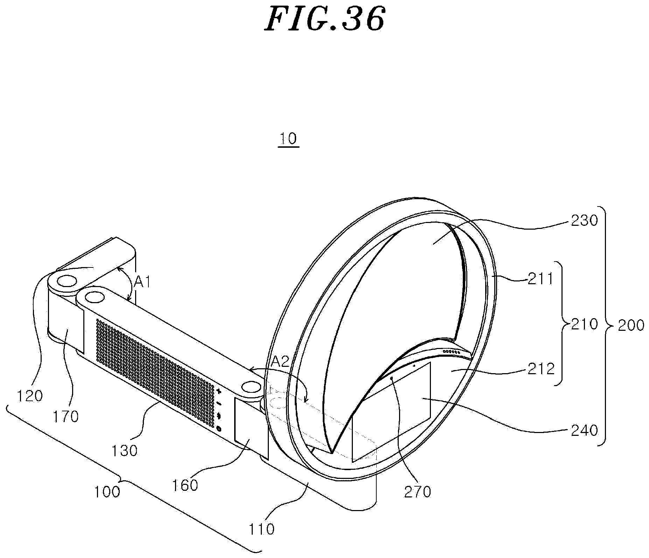

As shown in FIGS. 35 and 36, the voice recognition based smart mirror device 10 may further include an angle sensor 900, and the mirror unit 200 may further include a mirror motor 290.

The angle sensor 900 may detect arm angles between the plurality of arms 100 that are changed by rotating the plurality of arms 100 with respect to each other. For example, the angle sensor 900 detects a first arm angle A1 between the first arm 110 and the third arm 130, or a second arm angle A2 between the second arm 120 and the third arm 130. The first arm angle A1 may be increased or decreased as the first arm 110 and the third arm 130 rotate relative to each other such that they are folded or unfolded relative to each other. The second arm angle A2 may be increased or decreased as the second arm 120 and the third arm 130 rotate relative to each other such that they are folded or unfolded relative to each other.

The mirror motor 290 may rotate the mirror part 230 so that the mirror part 230 rotates with respect to the body part 210 about an axis extending in the up and down direction. The operation of the mirror motor 290 may be controlled by the controller 260. In particular, when the plurality of arms 100 are folded and the mirror unit 200 is in close contact with the wall surface in a state in which the mirror part 230 is rotated at a predetermined angle in the left and right direction with respect to the initial position of the body part 210, the mirror motor 290 may rotate the mirror part 230 to an initial position in which the mirror part 230 and the body part 210 are positioned on the same plane and in parallel so that the mirror part 230 is prevented from being damaged by colliding with the wall surface in advance.

The controller 260 may receive information about arm angles between the plurality of arms 100 from the angle sensor 900. When the plurality of arms 100 are rotated so that the arm angles between the plurality of arms 100 are smaller than a predetermined reference angle, the controller 260 may control the mirror motor 290 such that the mirror part 230 and the body part 210 are positioned on the same plane. Here, the predetermined reference angle may be a first reference angle between the first arm 110 and the third arm 130 and a second reference angle between the second arm 120 and the third arm 130, and at least one of the first reference angle and the second reference angle may be set as an acute angle.

For example, when the first reference angle is set to an acute angle, the first arm 110 is folded on the third arm 130 so that the first arm angle A1 between the first arm 110 and the third arm 130 becomes smaller than the first reference angle, the controller 260 may control the mirror motor 290 so that the mirror part 230 and the body part 210 are positioned on the same plane.

In addition, when the second reference angle is set to an acute angle, the third arm 130 is folded to the second arm 120 so that the second arm angle A2 between the third arm 130 and the second arm 120 is smaller than the second reference angle, the controller 260 may control the mirror motor 290 so that the mirror part 230 and the body part 210 are positioned on the same plane.

Accordingly, even when the plurality of arms 100 are folded and the mirror unit 200 is in close contact with the wall surface in a state in which the mirror part 230 is rotated at a predetermined angle in the left and right direction with respect to the body part 210, the mirror part 230 is located on the same plane as the body part 210 and does not directly collide with the wall surface so that the damage to the mirror part 230 can be prevented when the arm 100 is rotated.

Although the present disclosure has been described in detail with reference to the preferred embodiments, the scope of the present disclosure is not limited to specific embodiments, and should be interpreted based on the following claims. In addition, it will be understood by those skilled in the art that various changes and modifications can be made without departing from the scope of the present disclosure.

## Claims

1. A voice recognition based smart mirror device comprising:
a plurality of arms rotatably connected to each other;
a mirror unit rotatably mounted to one of the plurality of arms;
a speaker installed in at least one of the plurality of arms to output a sound; and
a bracket to be fixedly installed on a wall and configured to selectively mount one of the plurality of arms,
wherein the mirror unit includes:
a body part;
a joint part through which the body part is pivotably connected to the one of the plurality of arms;
a mirror part rotatably mounted to the body part;
a display module part mounted on a front surface of the body part to output an image, the display module part serving to sense a touch signal of a user;
a recognition part for detecting a voice signal of the user; and
a controller configured to control the display module part in accordance with the voice signal or the touch signal.

2. The voice recognition based smart mirror device of claim 1, wherein the mirror unit further includes: a display module part mounted on a front surface of the body part and configured to detect a touch signal of the user, and
wherein the controller is configured to control the display module part based on the touch signal.

3. The voice recognition based smart mirror device of claim 1 or 2, further comprising:
an actuator for rotating at least one of the plurality of arms,
wherein the actuator shifts a position of the mirror unit under the control of the controller to allow the mirror unit to be directed to a face of the user at various angles.

4. The voice recognition based smart mirror device of claim 3, wherein the actuator is linked to a voice assistant of the controller so that the position of the mirror unit is controlled in response to the voice signal of the user.

5. The voice recognition based smart mirror device of any one of claims 1 to 4, wherein the mirror unit further includes a mirror motor for rotating the mirror part with respect to the body part about an axis extending upward from the body part, and
the controller is configured to control the mirror motor so that the mirror part and the body part are positioned on the same plane when the plurality of arms are rotated so that arm angles between the plurality of arms are smaller than a predetermined reference angle.

6. The voice recognition based smart mirror device of claim 5, wherein the plurality of arms include a first arm, a second arm, and a third arm,
the predetermined reference angle includes a first reference angle between the first arm and the third arm and a second reference angle between the second arm and the third arm, and
at least one of the first reference angle and the second reference angle is an acute angle.

7. The voice recognition based smart mirror device of claim 5 or 6, further comprising:
an angle sensor for detecting arm angles between the plurality of arms that are changed when the plurality of arms are rotated with respect to each other.

8. The voice recognition based smart mirror device of any one of claims 1 to 7, wherein the body part comprises:
a body ring with a through hole in which the mirror part is mounted;
a body support provided under the body ring to support the display module part; and
a body shaft that connects the mirror part and the body support to allow the mirror part to rotate in the through hole.

9. The voice recognition based smart mirror device of claim 8, wherein a curved edge surface is formed at an upper end portion of the body support, the curved edge surface being inclined forward and curved along an edge of the body support so as to be convex upward.

10. The voice recognition based smart mirror device of any one of claims 1 to 5, wherein the plurality of arms comprises:
a first arm to which the mirror unit is mounted;
a second arm to be fixedly installed on a wall surface through a fixing bracket;
a third arm including a speaker therein and having a sound slit for outputting a sound of the speaker;
a first rotation shaft that connects one end portion of the first arm and one end portion of the third arm in an up-down direction to allow the first arm and the third arm to be rotated relative to each other; and
a second rotation shaft that connects one end portion of the second arm and the other end portion of the third arm in the up-down direction to allow the second arm and the third arm to be rotated relative to each other.

11. The voice recognition based smart mirror device of any one of claims 1 to 5, wherein the plurality of arms comprises:
a first arm to which the mirror unit is mounted;
a second arm to be fixedly installed on a wall surface through a fixing bracket;
a third arm including a speaker therein and having a sound slit for outputting a sound of the speaker;
a first link connecting one end portion of the first arm and one end portion of the third arm in a front-rear direction to link the first arm and the third arm; and
a second link connecting one end portion of the second arm and the other end portion of the third arm in the front-rear direction to link the second arm and the third arm.

12. The voice recognition based smart mirror device of any one of claims 2 to 11, insofar as dependent upon claim 2, wherein when the mirror unit is separated from the arm or docked to the arm in a state in which the display module part or the speaker outputs a sound, the controller controls the display module part and the speaker to maintain the output of the sound.

13. The voice recognition based smart mirror device of any one of the preceding claims, wherein the mirror unit further includes:
a request input part configured to receive a request from a user with respect to a biometric information measuring device for measuring biometric information of the user;
an artificial intelligence platform service module configured to recognize an operation corresponding to the received request among operations to be operated by the biometric information measuring device and generate a control command corresponding to the recognized operation; and
a transmitting part configured to transmit the generated control command to the biometric information measuring device.
